(19) Europäisches Patentamt / European Patent Office / Office européen des brevets

(11) **EP 4 468 025 A1**

(12) **EUROPEAN PATENT APPLICATION**
published in accordance with Art. 153(4) EPC

(43) Date of publication:
**27.11.2024 Bulletin 2024/48**

(21) Application number: **22922007.4**

(22) Date of filing: **06.10.2022**

(51) International Patent Classification (IPC):
***G01S 7/03*** (2006.01)

(52) Cooperative Patent Classification (CPC):
**G01S 7/03**

(86) International application number:
**PCT/JP2022/037501**

(87) International publication number:
**WO 2023/139854 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.01.2022 JP 2022006696**

(71) Applicant: **Kyocera Corporation**
**Kyoto-shi**
**Kyoto 612-8501 (JP)**

(72) Inventors:
• **MURAKAMI Youhei**
**Kyoto-shi, Kyoto 612-8501 (JP)**
• **KURODA Jun**
**Kyoto-shi, Kyoto 612-8501 (JP)**
• **SATO Masayuki**
**Kyoto-shi, Kyoto 612-8501 (JP)**
• **KAWAJI Satoshi**
**Kyoto-shi, Kyoto 612-8501 (JP)**
• **NAGASAWA Tadashi**
**Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC APPARATUS AND METHOD FOR DESIGNING ELECTRONIC APPARATUS**

(57) An electronic device includes a transmission antenna, a reception antenna, a cover part, and a signal processor. The transmission antenna is configured to transmit a transmission wave. The reception antenna is configured to receive a reflection wave resulting from reflection of the transmission wave. The cover part is configured to cover at least part of at least one of the transmission antenna or the reception antenna. The signal processor is configured to detect an object that reflects the transmission wave based on at least one of a transmission signal transmitted as the transmission wave or a reception signal received as the reflection wave. At least part of at least one of the transmission wave transmitted by the transmission antenna or the reflection wave received by the reception antenna is transmitted and/or received via the cover part. A thickness of the cover part is designed based on magnitude-squared coherence.

**EP 4 468 025 A1**

# FIG. 1

**Description**

CROSS-REFERENCE TO RELATED APPLICATIONS

This application claims priority of Japanese Patent Application No. 2022-006696 filed in Japan on January 19, 2022, the entire disclosure of which is hereby incorporated by reference.

TECHNICAL FIELD

The present disclosure relates to an electronic device and a method for designing an electronic device.

BACKGROUND OF INVENTION

In fields such as industries related to automobiles, technologies for measuring the distance between a host vehicle and a prescribed object are becoming increasingly important. In particular, in recent years, various studies have been conducted on RADAR (Radio Detecting and Ranging) technologies. In these technologies, the distance to an object, such as an obstacle, is measured by transmitting radio waves, such as millimeter waves, and receiving reflection waves reflected from the object. The importance of such technologies for measuring distances so forth is expected to further increase in the future with the development of technologies for assisting drivers with driving and technologies related to automated driving that allow part or all of the driving process to be automated.

Various technologies have been proposed for detecting, for example, the presence of an object by receiving reflection waves generated by transmitted radio waves being reflected off the object. For example, Patent Literature 1 proposes a device that can detect the presence of a person and the person's biometric information by using microwaves. For example, Patent Literature 2 proposes a device for detecting vital signs, such as the frequency of respiration or heartbeat of a living body, based on reflection signals of microwave radar. Furthermore, Patent Literature 3 discloses a radar device that achieves improved transmission-reception isolation as a result of a radome being provided with a partition wall that suppresses radio wave wraparound from a transmission antenna section to a reception antenna section.

CITATION LIST

PATENT LITERATURE

Patent Literature 1: Japanese Unexamined Patent Application Publication No. 2002-71825

Patent Literature 2: Japanese Unexamined Patent Application Publication No. 2021-32880

Patent Literature 3: Japanese Unexamined Patent Application Publication No. 2012-93305

SUMMARY

In an embodiment, an electronic device includes a transmission antenna, a reception antenna, a cover part, and a signal processor.

The transmission antenna is configured to transmit a transmission wave.

The reception antenna is configured to receive a reflection wave resulting from reflection of the transmission wave.

The cover part is configured to cover at least part of at least one of the transmission antenna or the reception antenna.

The signal processor is configured to detect an object that reflects the transmission wave based on at least one of a transmission signal transmitted as the transmission wave or a reception signal received as the reflection wave.

At least part of at least one of the transmission wave transmitted by the transmission antenna or the reflection wave received by the reception antenna is transmitted and/or received via the cover part.

A thickness of the cover part is designed based on magnitude-squared coherence.

In an embodiment, a method for designing an electronic device is provided.

The electronic device includes a transmission antenna, a reception antenna, a cover part, and a signal processor.

The transmission antenna is configured to transmit a transmission wave.

The reception antenna is configured to receive a reflection wave resulting from reflection of the transmission wave.

The cover part is configured to cover at least part of at least one of the transmission antenna or the reception antenna.

The signal processor is configured to detect an object that reflects the transmission wave based on at least one of a transmission signal transmitted as the transmission wave or a reception signal received as the reflection wave.

At least part of at least one of the transmission wave transmitted by the transmission antenna or the reflection wave received by the reception antenna is transmitted and/or received via the cover part.

The design method includes designing a thickness of the cover part based on magnitude-squared coherence.

In an embodiment, a method for designing an electronic device is provided.

The electronic device includes a transmission antenna, a reception antenna, and a signal processor.

The transmission antenna is configured to transmit a transmission wave.

The reception antenna is configured to receive a reflection wave resulting from reflection of the transmission wave.

The signal processor is configured to detect an object that reflects the transmission wave based on at least one of a transmission signal transmitted as the transmission wave or a reception signal received as the reflection wave.

The design method includes designing a prescribed specification in the electronic device based on magnitude-squared coherence.

BRIEF DESCRIPTION OF THE DRAWINGS

FIG. 1 is a perspective view for describing the configuration of an electronic device according to an embodiment.
FIG. 2 is a sectional view for describing the configuration of the electronic device according to an embodiment.
FIG. 3 is a block diagram illustrating the functional configuration of the electronic device according to an embodiment.
FIG. 4 is a flowchart for describing a method for designing for the electronic device according to an embodiment.
FIG. 5 is a diagram illustrating evaluation results of the electronic device including various cover parts according to an embodiment.
FIG. 6 is a diagram illustrating evaluation results of the electronic device including various cover parts according to an embodiment.
FIG. 7 is a block diagram illustrating the functional configuration of an evaluation system used when designing the electronic device according to an embodiment.
FIG. 8 is a diagram illustrating evaluation results of the electronic device including various cover parts according to an embodiment.
FIG. 9 is a diagram illustrating evaluation results of the electronic device including various cover parts according to an embodiment.
FIG. 10 is a diagram illustrating evaluation results of the electronic device including various cover parts according to an embodiment.
FIG. 11 is a flowchart for describing a method for designing the electronic device according to an embodiment.

DESCRIPTION OF EMBODIMENTS

An electronic device that can detect an object with good accuracy, such as a radar device including a suitable radome, is desired. In particular, electronic devices, such as radar devices, that can detect weak vibrations, such as the heartbeat of a human body or an animal, with good accuracy by transmitting and receiving radio waves, such as millimeter waves, are expected to be useful in a wide variety of fields. The present disclosure provides an electronic device that can detect objects with good accuracy, such as radar device including a suitable radome, and a method for designing such an electronic device. According to an embodiment, an electronic device that can detect objects with good accuracy, such as a radar device including a suitable radome, and a method for designing such an electronic device can be provided.

In the present disclosure, an "electronic device" may be a device that is driven by electric power. A "user" may be a person (typically a human) or an animal that uses an electronic device according to an embodiment. A user may also be a person who uses a method for designing an electronic device according to an embodiment. A user may also be a person who monitors a subject, such as a human, by using an electronic device according to an embodiment. A "subject" may be a person (for example, a human or an animal) to be monitored by an electronic device according to an embodiment. In addition, a user may be the subject. Furthermore, the subject is not limited to humans or animals, and may be any prescribed object. Here, a prescribed object may be, for example, a robot, a power tool, or audio equipment.

An electronic device according to an embodiment can detect the heartbeat of a subject, such as a human being, that is present in the vicinity of the electronic device. Therefore, possible locations where the electronic device according to an embodiment is used include, for example, a specific facility used by people engaged in social activities such as a company, a hospital, a senior citizens' home, a school, a gym, and a care facility. For example, understanding and/or managing the health status of employees and others in a company is extremely important. Similarly, understanding and/or managing the health status of patients, healthcare professionals, and others in a hospital, and residents, staff, and others in a senior citizens' home is extremely important. Locations where the electronic device according to an embodiment is used are not limited to facilities such as companies, hospitals, and senior citizens' homes, as described above, and may be any facility in which understanding and/or managing of the health status of subjects is desired. Any facility may also include non-commercial facilities such as a user's home. The locations where the electronic device according to an embodiment is used are not limited to indoors, and may also be outdoors. For example, locations where the electronic device according to an embodiment is used may be inside a mobile vehicle such as a train, a bus, and an airplane, as well as at a station and boarding area. Locations where the electronic device according to an embodiment is used may include a mobile vehicle such as an automobile, aircraft, or ship, a hotel, the user's own home, or the living room, bathroom, toilet, or bedroom of the user's own home.

The electronic device according to an embodiment may be used, for example, in a care facility to detect or monitor the heartbeat of a subject, such as a person in need of medical care or a person in need of assistance. The electronic device according to an embodiment may issue a prescribed warning to the person in question and/or other people, for example, when an abnormality is observed in the heartbeat of a subject, such as a person in need of medical care or a person in need of assistance. Therefore, the electronic device according to an embodiment allows the person in question and/or the staff of a care facility etc. to recognize that an abnormality is observed in the pulse rate of the subject such as a person in need of medical care or a person in need of assistance. On the other hand, the electronic device according to an embodiment may, for example, inform the person in question and/or other people of the fact when no abnormality is observed (e.g., observed to be normal) in the heartbeat of the subject such as a person in need of medical care or a person in need of assistance. Therefore, the electronic device according to an embodiment allows the person in question and/or the staff of a care facility to recognize that the pulse rate of the subject such as a person in need of medical care or a person in need of assistance is normal.

The electronic device according to an embodiment may detect the pulse rate of an animals other than a human. As an example, the electronic device according to an embodiment described below is described as a device that detects the pulse rate of a human by using a sensor based on a technology such as millimeter wave radar.

The electronic device according to an embodiment may be installed in any stationary object or in any mobile object. The electronic device according to an embodiment can transmit transmission waves from a transmission antenna to the surroundings of the electronic device. The electronic device according to an embodiment can receive reflection waves from a reception antenna, the reflection waves being generated by the transmission waves being reflected. At least one of the transmission antenna or the reception antenna may be included in the electronic device, for example, may be included in a radar sensor.

Hereafter, as a typical example, the electronic device according to an embodiment will be described as being stationary. On the other hand, the subject (human being) whose pulse rate is to be detected by the electronic device according to an embodiment may be stationary, moving, or moving his or her body while stationary. Similarly to an ordinary radar sensor, the electronic device according to an embodiment can measure a distance, such as the distance between the electronic device and an object in the surroundings of the electronic device, in a situation where the object is able to move. The electronic device according to an embodiment can, for example, measure a distance, such as the distance between the electronic device and an object, even when both the electronic device and the object are stationary.

Hereafter, an electronic device according to an embodiment will be described in detail while referring to the drawings. First, the configuration of the electronic device according to an embodiment will be described.

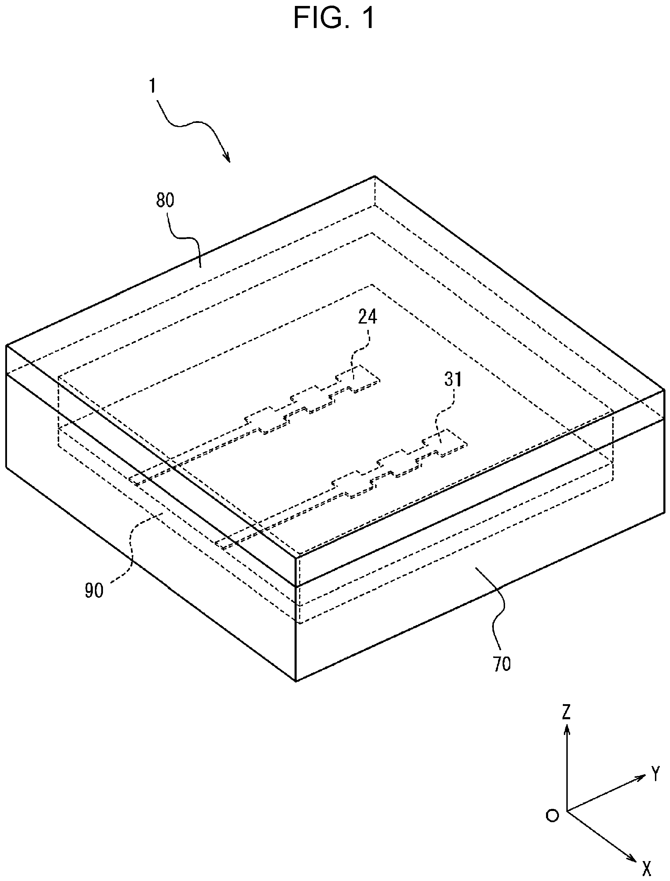

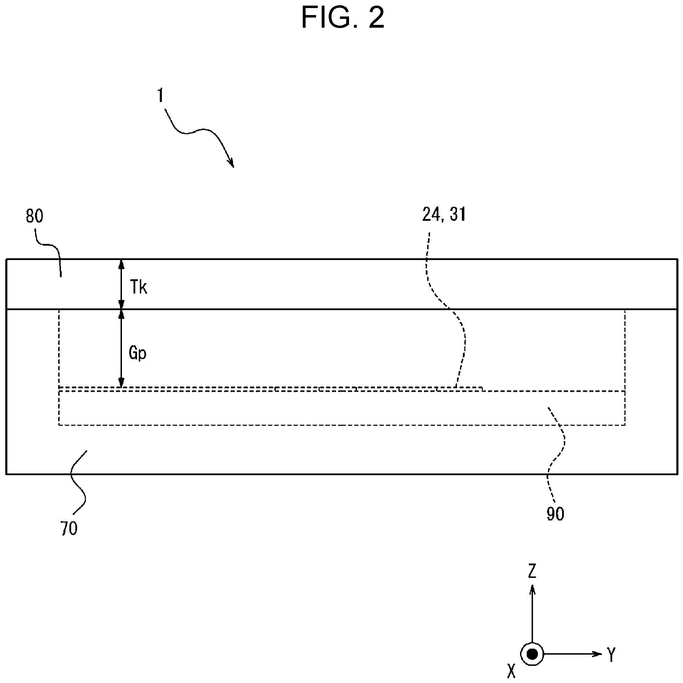

FIGs. 1 and 2 are diagrams for describing the configuration of the electronic device according to an embodiment. FIG. 1 is a perspective view of the electronic device according to an embodiment. FIG. 2 can be regarded as a sectional view or an end surface view of the electronic device according to an embodiment. Furthermore, both FIG. 1 and FIG. 2 illustrate the internal structure of the electronic device according to an embodiment in a see-through manner.

As illustrated in FIGs. 1 and 2, an electronic device 1 according to an embodiment may include a transmission antenna array 24, a reception antenna array 31, a housing 70, a cover part 80, and a substrate 90. The electronic device 1 illustrated in FIG. 1 represents an example of a case where there are one transmission channel and one reception channel. However, the electronic device 1 according to an embodiment may include any number of transmission channels and any number of reception channels.

The transmission antenna array 24 may be an array of antennas that transmit radio waves such as millimeter waves. The reception antenna array 31 may be an array of antennas that receive reflection waves generated by radio waves transmitted by the transmission antenna array 24 being reflected by, for example, a prescribed object. The transmission antenna array 24 and the reception antenna array 31 illustrated in FIG. 1 are each depicted as an array of three patch antennas. However, the transmission antenna array 24 and the reception antenna array 31 may each consist of any number of patch antennas as necessary. The transmission antenna array 24 and the reception antenna array 31 may be composed of a metal material such as copper.

As illustrated in FIGs. 1 and 2, the transmission antenna array 24 may transmit transmission waves including a positive Z-axis direction component. In addition, the reception antenna array 31 may transmit reflection waves including a negative Z-axis direction component.

As illustrated in FIGs. 1 and 2, the transmission antenna array 24 and the reception antenna array 31 may each be formed on the substrate 90. The substrate 90 may be configured using a low-loss dielectric material such as glass epoxy. The transmission antenna array 24 and the reception antenna array 31 may be disposed on the front surface of the substrate 90 (surface on positive Z-axis direction side). A circuit for generating a signal to be transmitted and/or a circuit for processing a signal to be received may be disposed on the rear surface of the substrate 90 (surface on negative Z-axis direction side).

The substrate 90 illustrated in FIGs. 1 and 2 is illustrated as having a quadrangular shape, for example, a square. However, in an embodiment, the substrate 90 may have any shape as needed. The substrate 90 is not necessarily limited to a flat plate-shaped member, and may be a curved member, for example. The thickness of the substrate 90 (in the Z-axis direction) is not particularly limited, and may be relatively thin, for example, around 1 mm to 2 mm. Furthermore, the size of the substrate 90 (in the XY directions) is not particularly limited, and may be relatively small, for example, around several centimeters.

The housing 70 may have a structure that protects at least any one of the transmission antenna array 24 and the reception antenna array 31 or the substrate 90, as illustrated in FIGs. 1 and 2. For example, the housing 70 may be configured to include a bottom surface and four side surfaces surrounding the bottom surface. The housing 70 may be of any size that allows at least any one of the transmission antenna array 24 and reception antenna array 31 or the substrate 90 to be accommodated. The housing 70 may be of any shape that allows the substrate 90 to be accommodated thereinside, for example. The housing 70 may be composed of a resin material such as ABS, acrylic, or PET (polyethylene terephthalate), for example.

The cover part 80 may cover the housing 70, which contains the transmission antenna array 24, the reception antenna array 31, and the substrate 90. The cover part 80 may be configured to cover at least part of at least one of the transmission antenna array 24 or the reception antenna array 31. Similarly to the housing 70, the cover part 80 may be composed of a resin material such as ABS, acrylic, or PET. The cover part 80 may be of any size such that at least part of at least one of the transmission antenna array 24 or the reception antenna array 31 can be covered. The cover part 80 may have a size designed in accordance with an opening of the housing 70.

The cover part 80 illustrated in FIGs. 1 and 2 is depicted as having a quadrangular shape, for example, a square shape. However, in an embodiment, the cover part 80 may have any shape as needed. The cover part 80 is not necessarily limited to a flat plate-shaped member, and may be a curved member, for example. In addition, the cover part 80 may include at least part of the housing 70 illustrated in FIGs. 1 and 2, for example. The thickness of the cover part 80 (in the Z-axis direction) is hereafter denoted as Tk, as illustrated in FIG. 2. The thickness Tk of the cover part 80 may be around 1 mm to 2 mm, for example. Furthermore, the size of the cover part 80 (in the XY directions) is not particularly limited, and may be relatively small, for example, around several centimeters.

The cover part 80 may be configured as a so-called radome. The radome functions to protect the antennas from the natural environment such as wind, rain, snow, sand, ice, and sunlight, while simultaneously visually concealing the antennas and/or the electronic device. The radome is typically composed of a material such as fiberglass or Teflon (registered trademark), which has high radio wave transmissivity. In the case of the electronic device 1 (planar radar device) as illustrated in FIGs. 1 and 2, the radome may be a planar cover member, for example. The radome may be a component depicted as only the cover part 80 of the electronic device 1 illustrated in FIGs. 1 and 2. The radome may include not only the cover part 80 of the electronic device 1 illustrated in FIGs. 1 and 2, but also at least part of the housing 70.

As illustrated in FIG. 2, a spacing Gp between at least one of the transmission antenna array 24 or the reception antenna array 31 disposed on the substrate 90 and the cover part 80 may be, for example, 2.42 mm. Here, the spacing Gp may be set to 2.42 mm based on the size of the half-wavelength ($\lambda/2$) of 62 GHz radio waves.

Hereafter, the electronic device 1 according to an embodiment will be described while assuming that the electronic device 1 is a radar device (radar sensor) based on millimeter wave radar technology. However, the electronic device 1 according to an embodiment is not limited to a millimeter-wave-radar radar device, and may be a radar device other than millimeter wave radar, for example. The electronic device 1 according to an embodiment is not limited to a radar device, and may also be a device based on LIDAR (Light Detection and Ranging, Laser Imaging Detection and Ranging) technology using light waves, for example. Furthermore, the electronic device 1 according to an embodiment may be a device based on a technology for detecting objects by transmitting and receiving sound waves or ultrasound waves, for example.

Frequency-modulated continuous wave radar (hereinafter referred to as FMCW radar) is often used when measuring distances using millimeter-wave-system radar. In FMCW radar, the transmission signal is generated by sweeping the frequency of the radio waves to be transmitted. Therefore, for example, in a millimeter-wave-system FMCW radar that uses radio waves in the 79 GHz frequency band, the frequency of the radio waves that are used have a frequency bandwidth of 4 GHz, for example, from 77 GHz to 81 GHz. Radar in the 79 GHz frequency band is characterized by having a wider usable frequency bandwidth than other millimeter/quasi-millimeter wave radars in the 24 GHz, 60 GHz, and 76 GHz frequency bands, for example.

The radar system of the FMCW radar used in this disclosure may include an FCM system (Fast-Chirp Modulation) that transmits chirp signals at a shorter period than normal. Signals generated by the electronic device 1 are not limited to FMCW-system signals. The signals generated by electronic device 1 may be signals of various systems other than the FMCW system. The transmission signal sequences stored in any storage unit may vary depending on these various systems. For example, in the case of radar signals of the FMCW system described above, a signal in which the frequency increases and a signal in which the frequency decreases in each time sample may be used. Since the various systems described above can be applied to known technologies as appropriate, more detailed description is omitted.

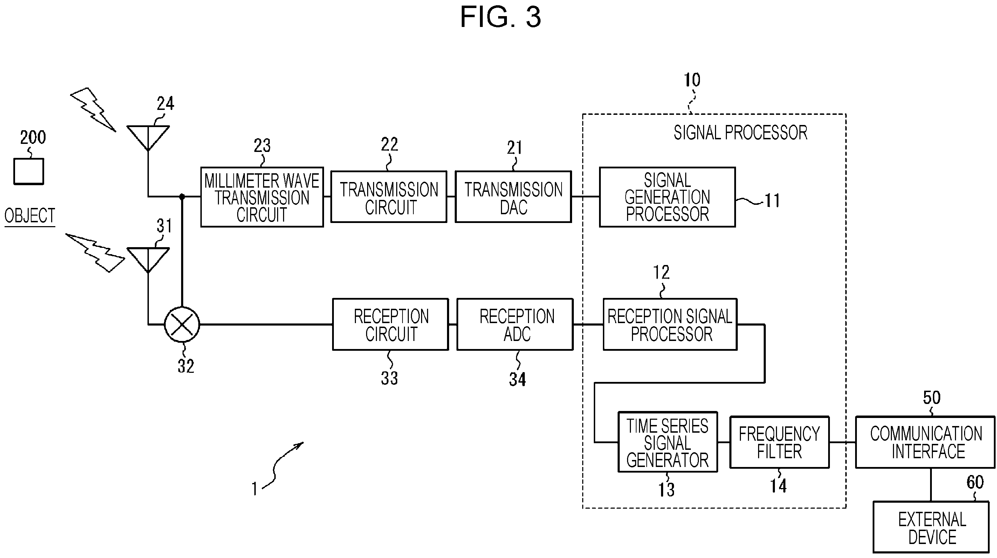

FIG. 3 is a block diagram illustrating the functional configuration of the electronic device 1 according to an embodiment. Hereafter, an example of the functional configuration of the electronic device 1 according to an embodiment will be described.

As illustrated in FIG. 3, the electronic device 1 according to an embodiment includes a signal processor 10. The signal processor 10 may include a signal generation processor 11, a reception signal processor 12, a time series signal generator 13, and a frequency filter 14. FIG. 3 illustrates the functional configuration of the electronic device 1 as described above. Therefore, among the functional units illustrated in FIGs. 1 and 2, the housing 70, the cover part 80, and the substrate 90 are not illustrated in FIG. 3.

Among the functional units illustrated in FIG. 3, the transmission antenna array 24 and the reception antenna array 31 may be located on the front surface (surface on positive Z-axis direction side) of the substrate 90 illustrated in FIGs. 1 and 2. In addition, at least some of the functional units illustrated in FIG. 3, other than the transmission antenna array 24 and the reception antenna array 31, may be located on the front surface (surface on positive Z-axis direction side) or the rear surface (surface on negative Z-axis direction side) of the substrate 90 illustrated in FIGs. 1 and 2. At least some of the functional units illustrated in FIG. 3, other than the transmission antenna array 24 and the reception antenna array 31, may be located inside or outside the housing 70 and the cover part 80 illustrated in FIGs. 1 and 2.

The electronic device 1 according to an embodiment includes a transmission DAC 21, a transmission circuit 22, a millimeter wave transmission circuit 23, and the transmission antenna array 24 as a transmission section. The electronic device 1 according to an embodiment includes the reception antenna array 31, a mixer 32, a reception circuit 33, and a reception ADC 34 as a reception section. The electronic device 1 according to an embodiment does not need to include at least any one of the functional units illustrated in FIG. 3 and may include functional units other than those illustrated in FIG. 3. The electronic device 1 illustrated in FIG. 3 may include circuits that are basically configured the same as or similar to those used in general radars that use electromagnetic waves in the millimeter wave band or the like.

The signal processor 10 of the electronic device 1 according to an embodiment can control of operation of the entire electronic device 1 including control of each functional unit included in the electronic device 1. In particular, the signal processor 10 performs various types of processing on signals handled by the electronic device 1. The signal processor 10 may include at least one processor, such as a CPU (central processing unit) or a DSP (digital signal processor), in order to provide control and processing power to perform various functions. The signal processor 10 may be implemented collectively in a single processor, in several processors, or in individual processors. The processors may be implemented as a single integrated circuit. An integrated circuit may also be referred to as an IC. Processors may be implemented as multiple integrated circuits and discrete circuits connected so as to be able to communicate with each other. The processors may be realized based on various other known technologies. In an embodiment, the signal processor 10 may be configured, for example, as a CPU (hardware) and a program (software) executed by the CPU. The signal processor 10 may appropriately include a storage unit (memory) as needed for the operation of signal processor 10.

The signal generation processor 11 of the signal processor 10 generates a signal to be transmitted from the electronic device 1. In the electronic device 1 according to an embodiment, the signal generation processor 11 may generate a transmission signal (transmission chirp signal), such as a chirp signal. In particular, the signal generation processor 11 may generate a signal whose frequency varies periodically and linearly (linear chirp signal). For example, the signal generation processor 11 may generate a chirp signal whose frequency periodically and linearly increases from 77 GHz to 81 GHz over time. For example, the signal generation processor 11 may generate a signal whose frequency periodically repeatedly linearly increases (up chirp) and decreases (down chirp) from 77 GHz to 81 GHz over time. The signal generated by the signal generation processor 11 may be set in advance in the signal processor 10, for example. The signal generated by the signal generation processor 11 may be stored in advance in any storage unit of the signal processor 10, for example. Since chirp signals used in technical fields such as radar are known, detailed description thereof is simplified or omitted as appropriate. The signal generated by the signal generation processor 11 is supplied to the transmission DAC 21. Therefore, the signal generation processor 11 (signal processor 10) may be connected to the transmission DAC 21.

The transmission DAC (digital-to-analog converter) 21 has a function of converting a digital signal supplied from the signal generation processor 11 into an analog signal. The transmission DAC 21 may include a general digital-to-analog converter. The signal generated by the analog conversion performed by the transmission DAC 21 is supplied to the transmission circuit 22. Therefore, the transmission DAC 21 may be connected to the transmission circuit 22.

The transmission circuit 22 has a function of converting the signal produced by the analog conversion performed by the transmission DAC 21 into a signal of an intermediate frequency (IF) band. The transmission circuit 22 may include a general IF band transmission circuit. A signal produced by processing performed by the transmission circuit 22 is supplied to the millimeter wave transmission circuit 23. Therefore, the transmission circuit 22 may be connected to the millimeter wave transmission circuit 23.

The millimeter wave transmission circuit 23 has a function of transmitting a signal produced by processing performed by the transmission circuit 22 as a millimeter wave (RF wave). The millimeter wave transmission circuit 23 may include a general millimeter wave transmission circuit. The signal produced by processing performed by the millimeter wave transmission circuit 23 is supplied to the transmission antenna array 24. Therefore, the millimeter wave transmission circuit 23 may be connected to the transmission antenna array 24. The signal produced by the processing performed by the millimeter wave transmission circuit 23 is also supplied to the mixer 32. Therefore, the millimeter wave transmission circuit 23 may also be connected to the mixer 32.

The transmission antenna array 24 is configured by arranging multiple transmission antennas in an array pattern. In FIG. 3, the configuration of the transmission antenna array 24 is illustrated in a simplified manner. The transmission antenna array 24 transmits the signal produced by processing performed by the millimeter wave transmission circuit 23 to outside the electronic device 1. The transmission antenna array 24 may include a transmission antenna array used in a general millimeter-wave radar.

Thus, the electronic device 1 according to an embodiment includes a transmission antenna (the transmission antenna array 24) and can transmit a transmission signal (for example, a transmission chirp signal) as a transmission wave from the transmission antenna array 24. As illustrated in FIGs. 1 and 2, the transmission wave transmitted from the transmission antenna array 24 may be transmitted to outside the electronic device 1 by passing through (being transmitted through) the cover part 80, such as a radome, for example.

For example, as illustrated in FIG. 3, a case in which an object 200 is present in the surroundings of the electronic device 1 is assumed. In this case, at least part of the transmission wave transmitted from the transmission antenna array 24 is reflected by the object 200. At least part of the wave reflected by the object 200, out of the transmission wave transmitted from the transmission antenna array 24, may be reflected towards the reception antenna array 31.

The reception antenna array 31 receives the reflection wave. Here, the reflection wave may be at least part of the wave reflected by the object 200 out of the transmission wave transmitted from the transmission antenna array 24.

The reception antenna array 31 is configured by arranging multiple reception antennas in an array pattern. In FIG. 3, the configuration of the reception antenna array 31 is illustrated in a simplified manner. The reception antenna array 31 receives a reflection wave resulting from reflection of the transmission wave transmitted from the transmission antenna array 24. As illustrated in FIGs. 1 and 2, the reception antenna array 31 may receive the reflection wave as described above from outside the electronic device 1 by the reflection wave passing through (being transmitted through) the cover part 80, such as a radome, for example.

The reception antenna array 31 may include a reception antenna array used in a typical millimeter-wave radar. The reception antenna array 31 supplies a reception signal received as a reflection wave to the mixer 32. Therefore, the reception antenna array 31 may be connected to the mixer 32.

The mixer 32 converts the signal produced by processing performed by millimeter wave transmission circuit 23 (transmission signal) and the reception signal received by reception antenna array 31 into a signal of an intermediate frequency (IF) bandwidth. The mixer 32 may include a mixer used in a general millimeter wave radar. The mixer 32 supplies the resulting combined signal to the reception circuit 33. Therefore, the mixer 32 may be connected to the reception circuit 33.

The reception circuit 33 has a function of analog processing the signal converted to an IF band by the mixer 32. The reception circuit 33 may include a typical reception circuit that converts a signal to an IF band. A signal produced by processing performed by the reception circuit 33 is supplied to the reception ADC 34. Therefore, the reception circuit 33 may be connected to the reception ADC 34.

The reception ADC (analog-to-digital converter) 34 has a function of converting an analog signal supplied from the reception circuit 33 into a digital signal. The reception ADC 34 may include a general analog-to-digital converter. A signal digitized by the reception ADC 34 is supplied to the reception signal processor 12 of the signal processor 10. Therefore, the reception ADC 34 may be connected to the signal processor 10 (reception signal processor 12).

The reception signal processor 12 of the signal processor 10 has a function of performing various types of processing on a digital signal supplied from the reception DAC 34. For example, the reception signal processor 12 can calculate the distance from the electronic device 1 to the object 200 (ranging) based on the digital signal supplied from the reception DAC 34. The reception signal processor 12 can calculate the velocity of the object 200 relative to the electronic device 1 (velocity measurement) based on the digital signal supplied from the reception DAC 34. The reception signal processor 12 can calculate the azimuth angle of the object 200 as seen from the electronic device 1 (angle measurement) based on the digital signal supplied from the reception DAC 34.

Specifically, I/Q converted data may be input to the reception signal processor 12. In response to input of the data, the reception signal processor 12 may perform a fast Fourier transform (2D-FFT) in distance (Range) and velocity (Velocity) directions, respectively. The reception signal processor 12 may then suppress false alarms and make the probability of false alarms constant by removing noise points through processing such as CFAR (Constant False Alarm Rate) processing. The reception signal processor 12 then obtains the position of the object 200 by, for example, performing arrival angle estimation for a point that satisfies the CFAR criterion. The information generated as results of the distance, velocity, and angle measurements performed by the reception signal processor 12 is supplied to the time series signal generator 13.

The time series signal generator 13 generates a time series signal representing the motion, for example, the vibration of the object 200, based on the information generated by the reception signal processor 12. As described above, the reception signal processor 12 can generate information of results obtained by measuring the distance, velocity, and angle of the object 200. Therefore, based on at least some of this information, the time series signal generator 13 can generate a time series signal representing vibration of the object 200, for example, when the object 200 is moving as though vibrating minutely. The time series signal representing the vibration of the object 200 generated by time series signal generator 13 may be supplied to the frequency filter 14.

The frequency filter 14 performs frequency filtering on the time series signal supplied from the time series signal generator 13. For example, the frequency filter 14 may perform processing to extract a micro-Doppler component. The frequency filter 14 may also perform processing to extract motion, such as vibration, of the object 200. Radar devices, such as millimeter wave radar, can measure the distance, angle, and velocity for multiple objects by using radio waves. Therefore, such radar devices can use the Doppler shift of received radio waves to detect the velocity of obj ects. Here, the Doppler shift changes when the object being detected is undergoing motion and/or vibration. The change in this Doppler shift over time is called a "micro-Doppler". By analyzing the micro-Doppler, information about the motion, such as vibration, of the object being detected can be obtained.

The information resulting from the filtering performed by the frequency filter 14 may be supplied to a communication interface 50, for example. Therefore, the frequency filter 14 (signal processor 10) may be connected to the communication interface 50. The resulting information output from the frequency filter 14 may be supplied to another functional unit other than the communication interface 50.

The communication interface 50 includes an interface that outputs information supplied from the signal processor 10 to an external device 60 for example. The communication interface 50 may output information of at least any one selected from among the position, velocity, and angle of the object 200 as a signal, for example, CAN (Controller Area Network), to the external device 60 or the like. For example, information of at least any one selected from among the position, velocity, and angle of the object 200 may be supplied to the external device 60 or the like via the communication interface 50. Information about the motion, such as vibration, of the object 200 described above may also be supplied through the communication interface 50 to the external device 60 or the like. Therefore, the communication interface 50 may be connected to the external device 60 or the like.

As illustrated in FIG. 3, the electronic device 1 according to an embodiment may be connected to the external device 60 in a wired or wireless manner via the communication interface 50. In an embodiment, the external device 60 may include any computer and/or any control device, etc. The electronic device 1 according to an embodiment may include the external device 60. The external device 60 can be configured in various ways depending on how the information about the motion, such as vibration, of the object 200 detected by the electronic device 1 is to be used. Therefore, detailed description of the external device 60 is omitted.

As described above, the electronic device 1 according to an embodiment transmits an electromagnetic wave as a transmission wave from the transmission antenna array 24. For example, when a prescribed object (for example, the object 200 illustrated in FIG. 3) is present in the surroundings of the electronic device 1, at least part of the transmission wave transmitted from the electronic device 1 will be reflected by the object and become a reflection wave. The reflection wave is, for example, received by the reception antenna array 31 of the electronic device 1, and this allows the electronic device 1 to detect the object as a target.

The object 200 may be, for example, a human present in the surroundings of the electronic device 1. The object 200 may also be a non-human living organism, such as an animal, that is present in the surroundings of the electronic device 1. Furthermore, the object 200 may be an object other than a living organism such as a human or animal. As described above, the object 200 may be moving, stopped, or stationary. In the present disclosure, objects detected by the electronic device 1 include inanimate objects such as any object, as well as living organisms such as people, dogs, cats, and horses, and other animals. Objects detected by the electronic device 1 of the present disclosure may include targets, including people, objects, and animals, to be detected by radar technology.

In an embodiment, the cover part 80 of the electronic device 1 can be designed by using information (for example, vibration waveforms) about the motion, such as vibration, of the object 200 supplied to the external device 60.

The design of the cover part 80 of the electronic device 1 according to an embodiment is further described below.

The electronic device 1 according to an embodiment can be realized as a device based on radar technology, such as a millimeter wave radar sensor that includes the transmission antenna array 24 and the reception antenna 31, as described above. The electronic device 1 according to an embodiment can also perform Doppler analysis using the distance to a detected object and a velocity component of the object. Here, when the electronic device 1 according to an embodiment includes the cover part 80 (and the housing 70) such as a radome, the electronic device 1 is configured so that the reflected power is suppressed in order to avoid degradation of the Doppler characteristics as much as possible. Therefore, a method for designing the electronic device 1 according to an embodiment relates to appropriately designing the cover member 80 (and the housing 70), such as a radome. The electronic device 1 according to an embodiment may include the cover member 80 (and the housing 70), such as a radome, appropriately designed by the design method as described above.

The electronic device 1 according to an embodiment can be realized as a highfrequency (e.g., 20 GHz or higher) Doppler radar for frequencies greater than or equal to a millimeter wave band that can perform distance measurement (ranging), angle estimation (angle measurement), and Doppler velocity detection using electromagnetic waves or sound waves etc. Therefore, the electronic device 1 according to an embodiment may be realized as a radar device using a frequency band of frequencies greater than or equal to millimeter waves that detects vibrations localized at a certain position as micro-Dopplers. In such a case, in the electronic device 1 according to an embodiment, the cover part 80 (and the housing 70), such as a radome, is preferably optimally designed.

Vibrations that can be detected by radar at frequencies greater than or equal to those of millimeter waves include, for example, vibrations of robots, power tools, and acoustic equipment, as well as movements of rigid bodies, and heartbeats, respiration, pulse rates, and movements of humans or animals. Generally, in order for a radar device to detect a micro-Doppler, the phase between IF signals corresponding to individual chirp signals, obtained through modulation, transmission, reception, and mixing using FCM or FMCW, needs to be accurately captured.

Therefore, the following two perspectives are important in order for the electronic device 1 according to an embodiment to detect vibrations such as micro-Dopplers. In other words,

First perspective: increase the isolation between the transmission antenna array 24 and the reception antenna array 31
Second perspective: reduce reflection caused by the cover part 80 (and the housing 70), such as a radome, of transmission waves transmitted from transmission antenna array 24 and/or reflection waves received by the reception antenna array 31

If we define the S-parameter (Scattering parameter) as 1 for a port of the transmission antenna array 24 and 2 for a port of the reception antenna array 31, the first and second perspectives described above can be expressed as follows. In other words,

First perspective: reduce $S_{21}$
Second perspective: reduce $S_{11}$ and/or $S_{22}$

In order to achieve the first perspective and the second perspective described above, the following design items are expected to be considered. In other words,

Design item 1: Design of antenna arrays and basic structure
Design item 2: Design of clearance and thickness of cover part 80
Design item 3: Refinement of design criteria through electromagnetic field analysis

These design items are further described below.

In design item 1, the directivity of antennas such as the transmission antenna array 24 and reception antenna array 31 may be controlled. Specifically, array design may be carried out in order to improve the performance of antenna arrays such as the transmission antenna array 24 and reception antenna array 31. The array design may be, for example, design of the far field and the near field by designing the number of arrays and the amplitude and phase coefficient for each channel (antenna) of the arrays, such as the transmission antenna array 24 and the reception antenna array 31.

In design item 2, a one-dimensional model may be used to design the following geometries based on the wavelength of a transmission wave transmitted from the transmission antenna array 24.

·The clearance between the transmission antenna array 24 and the reception antenna array 31 and the cover part 80 (spacing Gp illustrated in FIG. 2) is designed to be half the wavelength ($\lambda/2$) of the transmission wave in the air.
·The thickness of the cover part 80 (thickness Tk illustrated in FIG. 2) is designed to be half the wavelength of the transmission wave ($\lambda/2\sqrt{\varepsilon}$), taking into account the wavelength shortening rate due to the material constituting the cover part 80, such as a radome.

In design item 3, optimization may be performed by performing calculations based on an actual geometric model, taking into account the fact that the real-life arrangement of the transmission antenna array 24 and the reception antenna array 31 and the cover part 80 will extend in three dimensions. When performing calculations based on the actual geometric model, the transmission antenna array 24 and the reception antenna array 31 and the cover part 80 may be optimized by using electromagnetic field simulation or another method. FEM (Finite Element Method) and FDTD (Finite Difference Time, Domain method) may be used to perform electromagnetic field simulations.

However, when detection of vibration of the object 200 is to be performed, as in the electronic device 1 according to an embodiment, the above design alone is not expected to achieve sufficient phase accuracy between chirp signals. Therefore, in the electronic device 1 according to an embodiment, evaluation design focuses on phase accuracy between chirp signals. In other words, calculating the final vibration detection ability of the radar analytically or numerically by using design result information from the above design items 1 to 3 such as the S-parameter, far-field directivity, and near-field electromagnetic field distribution is difficult. Therefore, in the electronic device 1 according to an embodiment, the cover part 80 (and the housing 70) is designed by evaluating the design results using a system for evaluating vibration detection ability.

Thus, the electronic device 1 according to an embodiment may include the cover part 80 (and the housing 70) with the cover part 80 (and the housing 70) having been designed using a method for evaluating the detection ability of a radar using a vibrating body. In addition, an embodiment provides a method for designing the cover part 80 (and the housing 70) of the electronic device 1 by using a method for evaluating radar detection ability using a vibrating body.

Here, in the electronic device 1 according to an embodiment, the cover part 80 (and the housing 70) may be designed while taking the above design items 1 to 3 into consideration. For example, above-mentioned Patent Literature 3 (Japanese Unexamined Patent Application Publication No. 2012-93305) can be considered as focusing on the above design item 3. Patent Literature 3 proposes an experiment in which an attempt is made at improving isolation between transmission and reception antennas by installing a partition wall between the transmission and reception antennas.

Patent Literature 3 discloses that radar waves in the millimeter wave (76.5 GHz in this embodiment) band are transmitted and received in order to obtain information (distance, relative velocity, direction, etc.) about objects (vehicles in front, obstacles on the road, roadside objects, etc.) that reflect the radar waves. A front wall of a radome disclosed in Patent Literature 3 is provided with a partition wall that divides the internal space into two rectangular parallelepiped shaped spaces. The partition wall is integrally molded together with other parts of the radome. Here, the thickness of the radome, including the dividing wall, is disclosed as being formed to be about half the wavelength of the radar waves used (about 2 mm).

However, the following concerns are anticipated in the design described above.

In other words, the partition wall between the transmission and reception antennas significantly alters the near-field and far-field directivities of the antennas from the originally designed antenna array characteristics. Thus, even if this design improves the reception isolation, the antenna performance itself may be degraded. In addition, a radome (including the partition wall) designed as described above may not be able to ensure that the desired characteristics can be fully achieved in light of the first perspective and the second perspective described above.

Therefore, the cover part 80 (and housing 70) of the electronic device 1 according to an embodiment is designed using a system that evaluates the ability of the radar to detect vibrations in order to address the above-described concerns. According to the method of designing the electronic device 1 of an embodiment, the structure of the radome and/or housing can be designed so as to maximize the ability to detect vibrations. In other words, the electronic device 1 according to an embodiment can include a radome/housing structure that maximizes the vibration detection ability.

Hereafter, an example is described in which the thickness of the cover part 80 is optimized for when the spacing Gp from the transmission antenna array 24 and reception antenna array 31 to the cover part 80, as illustrated in FIG. 2, is 2.42 mm. Here, the spacing Gp may be set to 2.42 mm based on the size of the half-wavelength ($\lambda/2$) of 62 GHz radio waves.

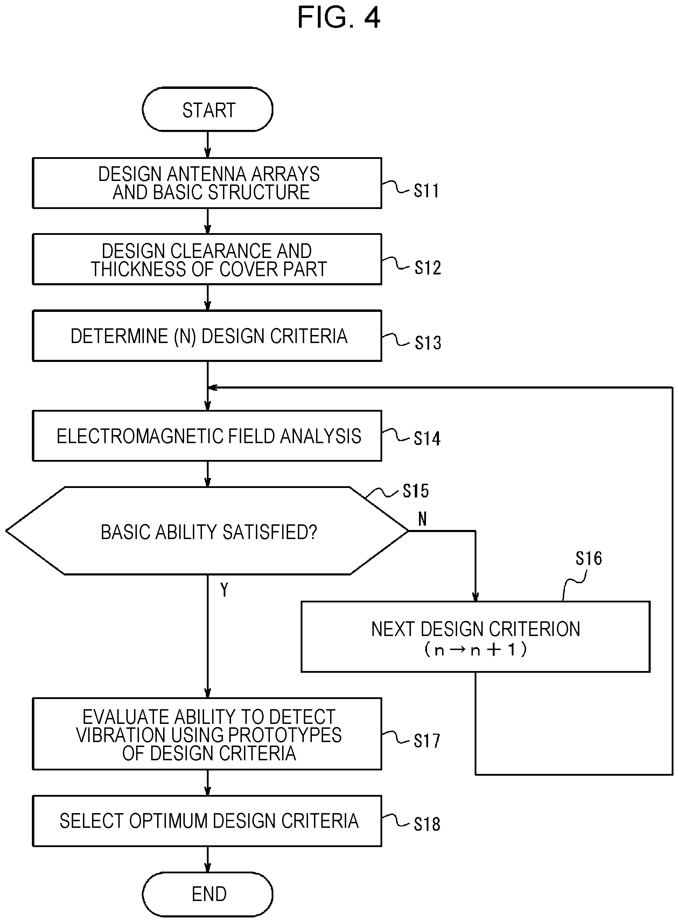

FIG. 4 is a flowchart illustrating a method of designing the cover part 80 of the electronic device 1 according to an embodiment. In an embodiment, the cover part 80 of the electronic device 1 can be designed by following the steps illustrated in the flowchart in FIG. 4.

When the design method illustrated in FIG. 4 is started, first, the above-mentioned design item 1, i.e., the antenna arrays and basic structure, is designed (Step S11). In Step S11, the directivities of antennas such as the transmission antenna array 24 and the reception antenna array 31 may be controlled as described above.

Next, design item 2 mentioned above, i.e., the clearance and the thickness of the cover part 80, is designed (Step S12). In Step S12, the spacing Gp (see FIG. 2) from the transmission antenna array 24 and the reception antenna array 31 to the cover part 80 may be designed as the clearance. In Step S12, the thickness Tk illustrated in FIG. 2 may be designed as the thickness of the cover part 80.

Steps S11 and S12 may be performed in the reverse order.

In an embodiment, N design criteria may be determined as a result of the design performed in Steps S11 and S12 (Step S13). In other words, at the time of Step S13, the design parameters of the electronic devices 1 with N different configurations may be determined.

Once the N design criteria have been determined, next, electromagnetic field analysis may be performed using each designed electronic device 1 (Step S14). In other words, in Step S14, electromagnetic field analysis may be performed for the N design criteria one after another. If a prescribed basic ability is determined to be satisfied (Yes in Step S15) as a result of performing the electromagnetic field analysis for a certain design criterion n ($\leq$ N) in Step S14, the process proceeds to Step S17. On the other hand, if the prescribed basic performance is determined to be not satisfied (No in Step S15) as a result of performing the electromagnetic field analysis for the certain design criterion n (≤ N) in Step S14, the electromagnetic field analysis in Step S14 may be performed for the next design criterion n+1 (Step S16). In this way, in Steps S14 to S16, the design criteria may be narrowed down based on the above-mentioned design item 3, i.e., electromagnetic field analysis.

In Step S15, whether or not the design criterion satisfies the basic capability based on the S-parameter and/or the realized gain may be determined. Hereafter, an example of refinement of design criteria by the electromagnetic field analysis performed in Steps S14 to S16 is further described.

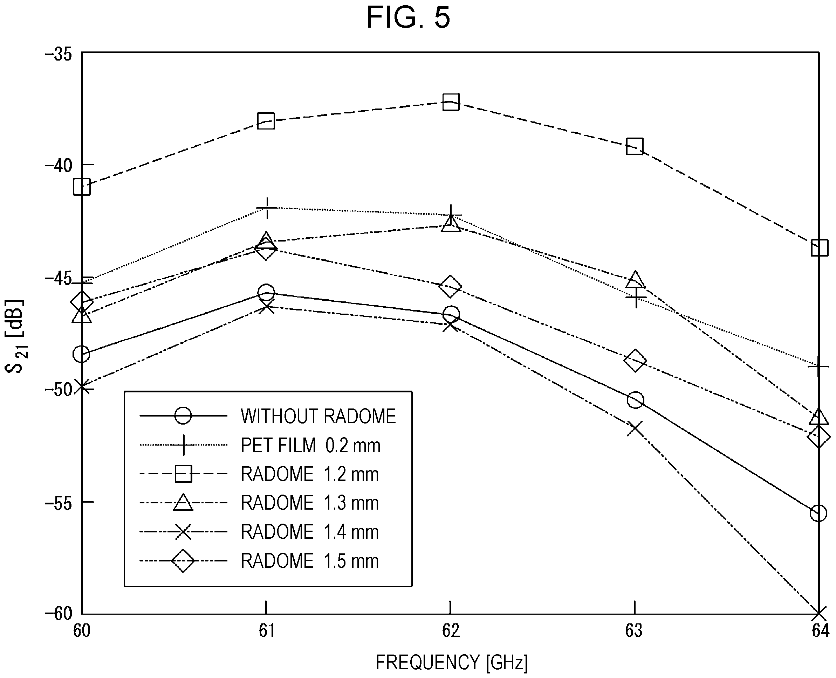

FIG. 5 illustrates an example of the electromagnetic field analysis performed in Step S14. FIG. 5 illustrates an example of electromagnetic field calculation results (finite element method) illustrating the degree to which the isolation between the transmission antenna array 24 and the reception antenna array 31 depends on the cover part 80. In other words, FIG. 5 is a diagram illustrating whether or not $S_{21}$, which corresponds to the first perspective described above, is reduced when the thickness Tk of the cover part 80 is changed to several values.

The horizontal axis in FIG. 5 represents the frequency of the transmission wave, and the vertical axis in FIG. 5 represents $S_{21}$, which corresponds to the first perspective described above. That is, the vertical axis in FIG. 5 represents the reflected power from port 1 to port 2, and illustrates that the smaller this value is, the better the characteristic. In FIG. 5, an example is illustrated in which the cover part 80 (radome) is composed of ABS resin. The thickness Tk of the cover part 80 (radome) was varied from 1.0 mm to 1.6 mm and a PET film was set as 0.2 mm, and six good cover parts 80 were selected and calculated values therefor are listed.

Considering the fact that the relative permittivity of ABS resin is 3.4, the wavelength is 1.31 mm when the transmission wave is 62 GHz. Therefore, a design criterion is set as ±0.3 mm before and after that wavelength. A very thin PET film is also set as a design criterion in FIG. 5 due to the high potential thereof to transmit radio waves. In addition, for reference, a case without a radome is also illustrated in FIG. 5. In the example illustrated in FIG. 5, the best results are obtained when the thickness Tk of the cover part 80 (radome) is 1.4 mm, rather than when there is no cover part 80 (radome). However, the best design result cannot be determined from this result alone.

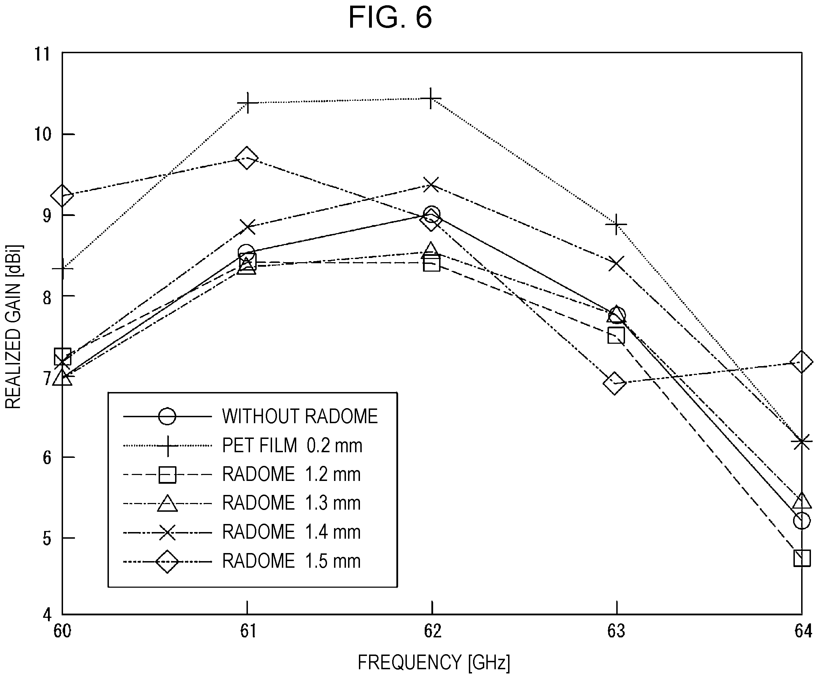

FIG. 6 illustrates the degree of realized gain for the electronic device 1 when the thickness Tk of cover part 80 is changed to several values. The horizontal axis in FIG. 6 represents the frequency of the transmission waves and the vertical axis in FIG. 5 represents realized gain. That is, the vertical axis in FIG. 6 illustrates that higher values indicate higher gain.

As illustrated in FIG. 6, when the thickness Tk of the cover part 80 (radome) is the 0.2 mm PET film, the gain is higher than that without the cover part 80 (radome). As illustrated in FIG. 6, the next highest gain to when the thickness Tk of the cover part 80 (radome) is a 0.2 mm PET film is obtained when the thickness Tk of the cover part 80 (radome) is 1.4 mm. On the other hand, we can see that when the thickness Tk of the cover part 80 (radome) is 1.5 mm, the frequency response is different from that in the other cover parts 80. In this case, the gain was particularly lowest in the 63 GHz band.

As can be seen from the results of FIGs. 5 and 6, the design criterion for which the isolation between transmitter and receiver is lowest and the design criterion for which the realized gain is best are not necessarily the same. Therefore, the optimal condition for the thickness Tk of the cover part 80 (radome) cannot be calculated only by narrowing down the design criteria through electromagnetic field analysis in Steps S14 to S16. Therefore, in an embodiment, the narrowing down of design criteria by electromagnetic field analysis in Steps S14 to S16 may be used only as a reference condition for narrowing down the design criteria.

Therefore, in an embodiment, prototypes of the electronic device 1 that satisfy M (1 ≤ M ≤ N) design criteria narrowed down as a result of Step S15 illustrated in FIG. 4 may be fabricated, and Steps S17 and S18 may be performed for the prototype electronic devices 1. In Step S17, prototypes of the electronic device 1 that satisfy the design criteria that passed Steps S14 and S15 are used to evaluate the ability to detect vibrations of the object 200. Once the evaluation of the ability to detect vibrations has been performed in Step S 17, the optimal design criteria for the electronic device 1 can be selected based on the results of the evaluation (Step S18).

The evaluation of the ability to detect vibrations of the object 200 performed in Step S17 may be, for example, a comparison of magnitude-squared coherence (MSC) using a vibrating body and a reference machine (instrument). As a result of this comparison, the electronic device 1 with optimal conditions can be selected. Hereafter, comparison of magnitude-squared coherence (MSC) using a vibrating body and reference machine (instrument) instrumentation is further described.

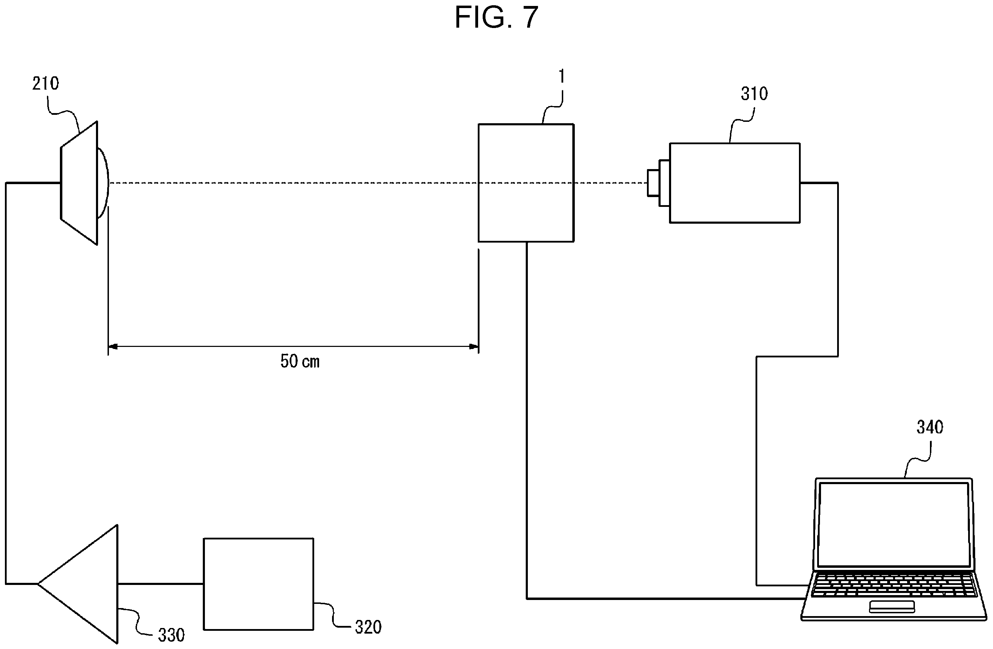

FIG. 7 illustrates the schematic configuration of an evaluation system for evaluating the vibration detection ability (hereinafter simply referred to as "evaluation system") illustrated in Step S17 of FIG. 4.

As illustrated in FIG. 7, the evaluation system may include a vibration source 210, a reference machine 310, a signal generator 320, an amplifier 330, and a data logger 340, in addition to the electronic device 1 that is to be evaluated.

The electronic device 1 illustrated in FIG. 7 may be, for example, the electronic device 1 fabricated as a prototype to satisfy the M (1 ≤ M ≤ N) design criteria narrowed down as a result of Step S15 illustrated in FIG. 4. The vibration source 210 may include a vibrating object as the object 200 illustrated in FIG. 3. In an embodiment, the vibration source 210 may include, for example, a loudspeaker that converts electrical vibrations into physical vibrations. The reference machine 310 may be a laser vibrometer, for example, in an embodiment. The signal generator 320 generates a test signal output from the vibration source 210. The amplifier 330 may include, for example, an audio amplifier that amplifies the test signal output from vibration source 210. The data logger 340 logs data output from the electronic device 1 and the reference machine 310.

The signal generated by the signal generator 320 illustrated in FIG. 7 may be a chirp signal of 500 Hz or less, for example. The dynamic range of the signal generated by the signal generator 320 may be from -30 dB to 0 dB, for example, with the level gradually decreasing by 3 dB per unit time. Such a signal allows the vibration amplitude of the vibration source 210 (loudspeaker) to be changed from a high state to a low state in order to verify at what level detection of the signal of the electronic device 1 signal reaches its limit. The distance between the vibration source 210 and the electronic device 1 was 50 cm, as illustrated in FIG. 7.

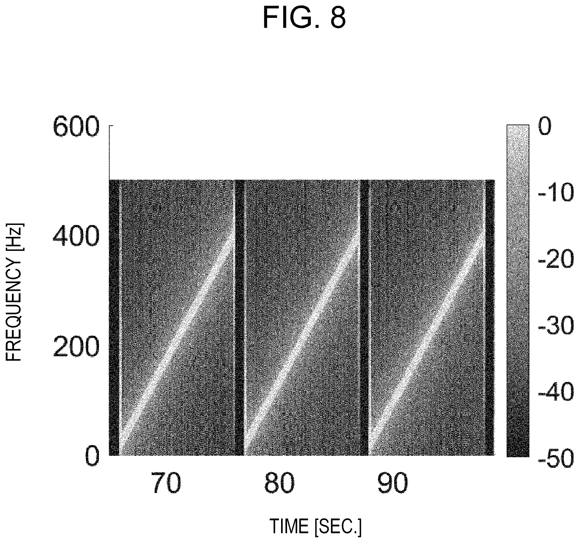

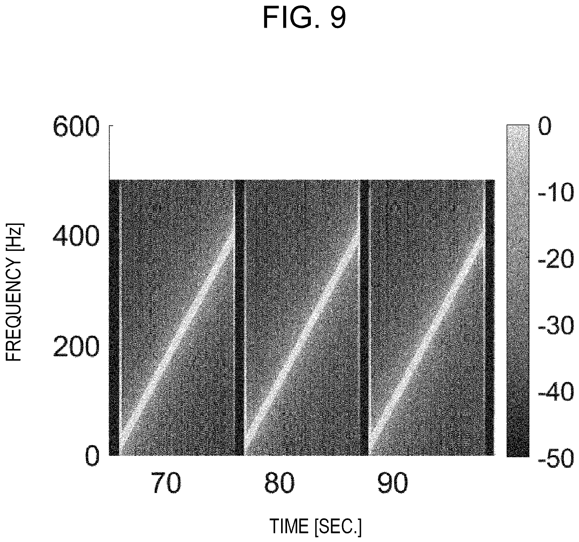

FIGs. 8 and 9 illustrate examples of signals input from the signal generator 320 to the vibration source 210 in the evaluation system as illustrated in FIG. 7. FIG. 8 illustrates the waveform of the entire time series of the signal. The horizontal axis in FIG. 8 represents time and the vertical axis in FIG. 8 represents signal level. FIG. 9 illustrates a one-level spectrogram. The horizontal axis in FIG. 9 represents time, and the vertical axis in FIG. 9 represents frequency. The results illustrated in FIGs. 8 and 9 allow us to confirm characteristics of the signal as described above.

Based on the evaluation system illustrated in FIG. 7 and the signals illustrated in FIGs. 8 and 9, the quality of the vibration data of the object 200, for example, detected by the electronic device 1 with respect to the reference machine 310 can be examined. The statistics to be examined are described below.

In the evaluation system illustrated in FIG. 7, magnitude-squared coherence $C_{xy}$ using the vibration source 210 and reference machine 310 can be expressed as in Equation (1) below. Here, f [Hz] is the frequency of the signal illustrated in FIG. 8 and FIG. 9 (audio signal). x is a time-series signal vector of a vibration detected by the reference machine 310 and y is a time-series signal vector of a vibration detected by the electronic device 1. $P_{xx}(f)$ and $P_{yy}(f)$ are respective power spectral densities of x and y.
[Math 1]

$$C_{xy} = \frac{|P_{xy}(f)|^2}{P_{xx}(f)P_{yy}(f)} \qquad (1)$$

By averaging the magnitude-squared coherence $C_{xy}$ as expressed in Equation (1) above within a prescribed frequency band, the final statistics as expressed in Equation (2) below can be obtained.
[Math 2]

$$\bar{C}_{xy} = \frac{1}{f_1 - f_0} \int_{f_0}^{f_1} C_{xy}(f) df \qquad (2)$$

The statistics calculated using Equation (2) above may be integrated for the design criteria narrowed down by the electromagnetic field analysis illustrated in Step S14 in FIG. 4, with $f_0$ = 20 [Hz] and $f_1$ = 400 [Hz].

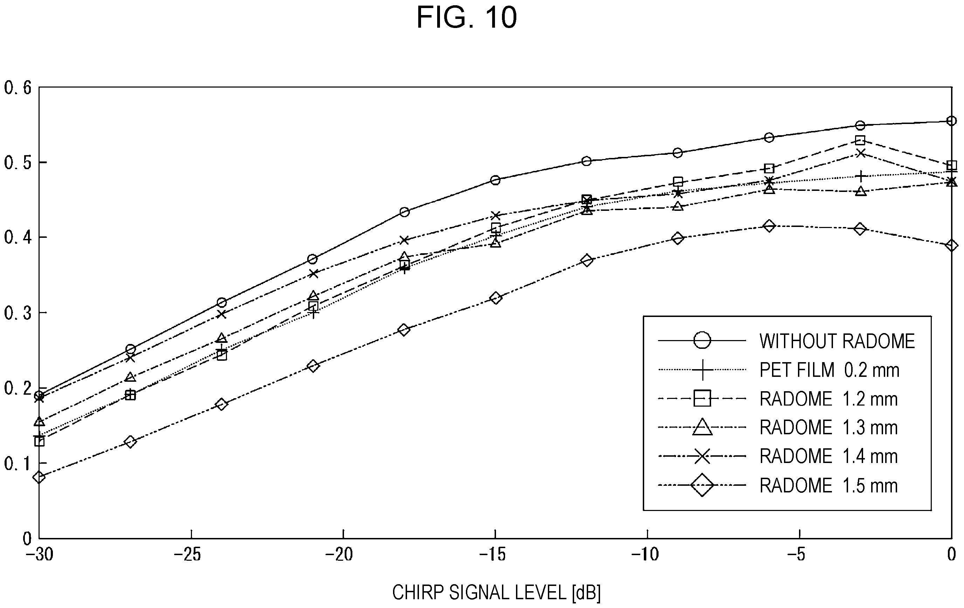

FIG. 10 is a diagram summarizing the above results for several design criteria of the prototype electronic device 1. The horizontal axis in FIG. 10 represents the chirp signal level and the vertical axis in FIG. 10 represents the averaged magnitude-squared coherence.

As can be seen from FIG. 10, the electronic device 1 configured as described above has the highest averaged magnitude-squared coherence when the cover part 80 (radome) is not present. The electronic device 1 has the highest averaged magnitude-squared coherence when the signal level is from -12 to -30 dB and when the thickness Tk of the cover part 80 (radome) is 1.4 mm. The electronic device 1 can be said to have a relatively higher averaged magnitude-squared coherence when the thickness Tk of the cover part 80 (radome) is 1.4 mm than for the other cases, even when the signal level is from 0 to -12 dB. On the other hand, the averaged magnitude-squared coherence is lower at all signal levels for 1.5 mm. Therefore, in the electronic device 1 configured as described above, the ability to detect vibration can be maximized by setting the thickness Tk of the cover part 80 (radome) to 1.4 mm.

Therefore, the method of designing the electronic device 1 according to an embodiment allows the thickness Tk of the cover part 80 (radome) of the electronic device 1 to be optimized. The thus-designed electronic device 1 according to an embodiment can detect objects (particularly the motion, such as vibration, of objects) with good accuracy as a result of the thickness Tk of the cover part 80 (radome) of the electronic device 1 being optimized.

(Other Embodiments)

Other embodiments are described below.
In another embodiment, rather than performing all of the steps illustrated in FIG. 4, at least some of the steps may be omitted, as appropriate. For example, in another embodiment, the electromagnetic field analysis may be omitted by skipping Steps S14 to S16 illustrated in FIG. 4.

In another embodiment, the vibration source 210 illustrated in FIG. 5 is not limited to a loudspeaker, and may be any transducer capable of generating planar vibrations, such as a piezoelectric transducer or an electrostatic speaker/actuator.

In another embodiment, the signal input to the vibration source 210, as illustrated in FIG. 8 and FIG. 9, is not limited to a chirp signal, and may be a frequency step signal that varies discretely with each frequency step, for example.

Other embodiments are not limited to designing only the thickness Tk of the cover part 80 (radome) of the electronic device 1 according to an embodiment. For example, other embodiments may relate to the design of any hardware of equipment that detects vibrations, such as the design of the entire housing 70, the transmission antenna array 24, and/or the reception antenna array 32.

The chirp signal input to the vibration source 210, as illustrated in FIG. 8 and FIG. 9, may be, for example, a step tone signal or a TSP (time stretched pulse) signal.

The bandwidth and/or dynamic range of the signal input to the vibration source 210, as illustrated in FIG. 8 and FIG. 9, may be freely set.

In Equation (2) above, the average within the frequency band was calculated. However, in other embodiments, another statistical value may be used, such as the statistical median, maximum, or minimum. When calculating an average as in Equation (2) above, a weighting factor w(f) may be multiplied in order to weight the bands that are important as frequencies, for example, as in Equation (3) below.

[Math 3]

$$\bar{C}_{xy} = \frac{1}{f_1 - f_0} \int_{f_0}^{f_1} w(f) C_{xy}(f) df \qquad (3)$$

(Design Criteria to be Considered)

In the embodiment described above, the design of the cover part 80 (and housing 70), such as a radome, was considered (and optimized) in order to ensure good accuracy of object detection by an electronic device such as a radar device. In particular, in the embodiment described above, optimization of the design of the clearance (spacing Gp) between the transmission antenna array 24 and reception antenna array 31 and the cover part 80 and/or the design of the thickness of the cover part 80 (thickness Tk) was described. On the other hand, the above-described embodiment is not limited to the optimization of the design of the spacing Gp and/or the design of the thickness Tk. The above-described embodiment is also not limited to optimization of the design of cover part 80 and/or the design of the housing 70. In other words, in the above-described embodiment, the design criteria to be considered are not limited to the size of the spacing Gp and/or the thickness Tk, or to the cover part 80 and/or housing 70.

As described above, for a radar device to detect micro-Dopplers, the phase between IF signals corresponding to individual chirp signals, obtained through modulation, transmission, reception, and mixing using FCM or FMCW, needs to be accurately captured. However, in radar devices, noise may be generated and signals may be degraded due to the various hardware and/or software, etc. used. Consequently, accurately capturing the phase between IF signals corresponding to individual chirp signals as described above is difficult. In this case, since the accuracy of the phase between chirps is degraded, accurately detecting a micro-Doppler signal is difficult. Therefore, in the embodiment described above, the spacing Gp and/or thickness Tk were considered as design criteria targets based on the first perspective (reducing $S_{21}$) and the second perspective (reducing $S_{11}$ and/or $S_{22}$).

On the other hand, if targets other than the spacing Gp and/or thickness Tk are changed as design criteria targets, noise may also be generated or signal degradation may also occur due to the various hardware and/or software used. Therefore, the accuracy with which the radar device detects objects can be made even better by also considering targets other than the spacing Gp and/or the thickness Tk mentioned above as design criteria targets. Hereafter, a case in which targets other than the spacing Gp and/or the thickness Tk described above are also included in the design criteria targets and considered is described.

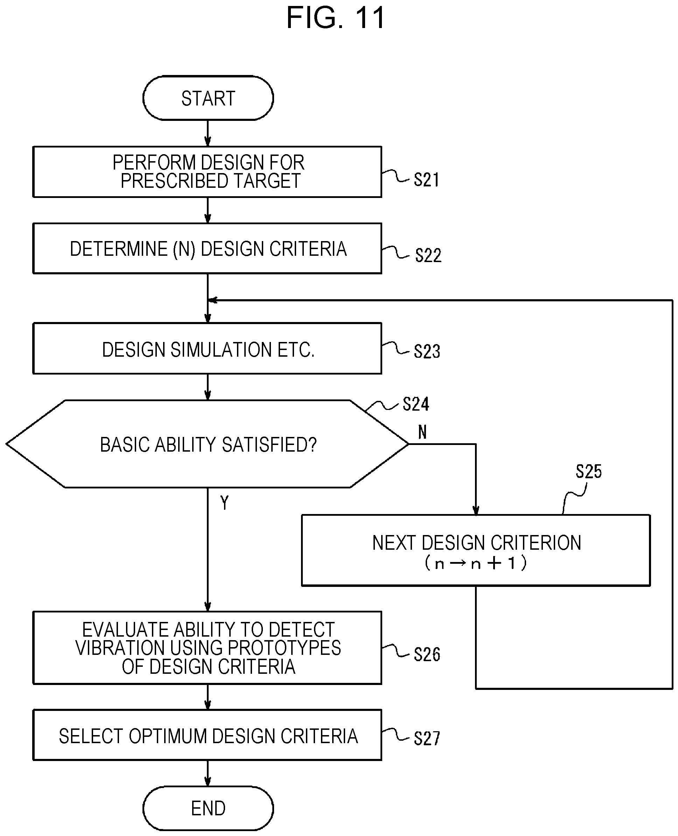

FIG. 11 is a flowchart for describing a method of designing the electronic device 1 according to an embodiment. FIG. 11 is a diagram illustrating a design method in which targets other than the cover part 80 of the electronic device 1 are included in the design criteria targets in the method described in FIG. 4. In FIG. 11, descriptions that are the same or similar to those already described for FIG. 4 will be simplified or omitted as appropriate. In an embodiment, the electronic device 1 can be designed by following the steps illustrated in the flowchart in FIG. 11.

When the design method illustrated in FIG. 11 starts, first, design is performed for prescribed targets as design criteria targets in the electronic device 1 (Step S21). The prescribed targets to be designed in Step S21 may be various targets based on various perspectives in the electronic device 1. For example, a prescribed target may be the shape, size, material, or structure of the cover part 80 and/or the housing 70. A prescribed target may be, for example, the number of channels of the transmission antenna array 24 and/or reception antenna array 31. A prescribed target may be, for example, the number, shape, size, material, or arrangement of the antenna elements constituting the transmission antenna array 24 and/or reception antenna array 31. A prescribed target may be, for example, the distance between the antenna elements constituting the transmission antenna array 24 and/or reception antenna array 31, the way in which the antenna elements are connected to each other, the number of feed points feeding the antenna elements, or the distances to the feed points feeding the antenna elements. Other prescribed targets may be various targets regarding the hardware and/or software constituting the electronic device 1. Prescribed targets may be various targets that can affect noise in the electronic device 1.

In Step S21, the antenna arrays and basic structure may be designed as in Step S11 illustrated in FIG. 4. In Step S21, the clearance (the spacing Gp from the transmission antenna array 24 and reception antenna array 31 to the cover part 80) and the thickness of the cover part 80 (thickness Tk) may be designed as in Step S12 illustrated in FIG. 4. In other words, in Step S21, design may be performed based on perspectives that include at least part of design Item 1 and/or design Item 2 described above.

In an embodiment, for example, N design criteria may be determined (Step S22) as a result of the design performed in Step S21. In other words, at the time of Step S22, design parameters of the electronic device 1 with N different configurations may be determined.

Once the N design criteria have been determined, next, prescribed design simulation may be performed using each designed electronic device 1 (Step S23). In other words, in Step S23, a prescribed design simulation may be performed for the N design criteria one after another.

The prescribed design simulation performed in Step S23 may be a simulation to determine whether or not a basic prescribed ability is satisfied for the prescribed target designed in Step S21. For example, let us suppose that the size of the cover part 80 was designed as a prescribed target in Step S21. In this case, N different sizes may be determined for the cover part 80 of the electronic device 1 in Step S22. Then, in Step S23, design simulations for the size of the cover part 80 may be performed for the N different sizes of the cover part 80 in the electronic device 1 one after another. The design simulation performed here may be a simulation in which the design of the size of the cover part 80 is simulated in order to determine whether any one of the N different sizes of the cover part 80 will satisfy the basic prescribed ability. The electromagnetic field analysis performed in Step S14 illustrated in FIG. 4 may be a specific example of the prescribed design simulation performed in Step S23.

If the prescribed basic ability is determined to be satisfied (Yes in Step S24) as a result of performing the design simulation for a certain design criterion n (≤ N) in Step S23, the process proceeds to the operation of Step S26. On the other hand, if the prescribed basic ability is determined to be not satisfied (No in Step S24) as a result of performing the electromagnetic field analysis for the certain design criterion n (≤ N) in Step S23, the design simulation in Step S23 may be performed for the next design criterion n+1 (Step S25). In this way, the design criteria may be narrowed down by performing design simulation in Steps S23 to S25.

In Step S24, whether or not the design criterion satisfies the basic ability based on the S-parameter and/or the realized gain may be determined, for example, as described in Step S15 in FIG. 4.

Next, prototypes of the electronic device 1 that satisfy M (1 ≤ M ≤ N) design criteria narrowed down as a result of Step S24 may be fabricated, and Steps S26 and S27 may be performed for the prototype electronic devices 1. In Step S26, prototypes of the electronic device 1 that satisfy the design criteria that passed Steps S23 and S24 are used to evaluate the ability to detect vibrations of the object 200. Once evaluation of the ability to detect vibrations has been performed in Step S26, the optimal design criteria for the electronic device 1 can be selected based on the results of the evaluation (Step S27).

The evaluation of the ability to detect vibrations of the object 200 performed in Step S26 may be, for example, a comparison of magnitude-squared coherence (MSC) using a vibrating body and a reference machine (instrument), the same as or similar to Step S17 in FIG. 4. As a result of this comparison, the electronic device 1 with optimal conditions can be selected.

In Step S26, the evaluation system as illustrated in FIG. 7 may be used to evaluate the ability to detect vibrations of the object 200, the same as or similar to Step S17 described in FIG. 4.

The electronic device 1 illustrated in FIG. 7 may be, for example, the electronic device 1 fabricated as a prototype to satisfy the M (1 ≤ M ≤ N) design criteria narrowed down as a result of Step S24 illustrated in FIG. 11. The vibration source 210 may include a vibrating object as the object 200 illustrated in FIG. 3. In an embodiment, the vibration source 210 may include, for example, a loudspeaker that converts electrical vibrations into physical vibrations. The reference machine 310 may be a laser vibrometer, for example, in an embodiment.

On the other hand, in an embodiment, the reference machine 310 may be a device other than a laser vibrometer. In an embodiment, the reference machine 310 may be, for example, a single microphone that collects sound or a voice, or a microphone array that detects sound or a voice in a prescribed direction. In an embodiment, the reference machine 310 may be a piezoelectric vibration sensor (for example, a piezosensor) that detects vibration by being in contact with a detection target. In this case, the reference machine 310 illustrated in FIG. 7 may be disposed so as to contact the vibration source 210, rather than being disposed away from the vibration source 210. In an embodiment, the reference machine 310 may employ a configuration based on a PPSI method (Parallel Phase-Shifting Interferometry) using a high-speed camera and a sound field optical measurement method.

As described above, in an embodiment, the accuracy with which the radar device detects objects may be improved by also considering targets other than the spacing Gp and/or the thickness Tk described above as design criteria targets.

Thus, the method for designing the electronic device 1 according to an embodiment may include a step of designing a prescribed specification in electronic device 1 based on magnitude-squared coherence.

In an embodiment illustrated in FIG. 11, at least some of the steps illustrated in FIG. 11 may be omitted, as appropriate, rather than performing all of the steps. For example, in the embodiment illustrated in FIG. 11, the design simulation may be omitted by skipping Steps S23 to S25.

The present disclosure has been described based on the drawings and examples, but note that a variety of variations and amendments may be easily made by one skilled in the art based on the present disclosure. Therefore, note that such variations and amendments are included within the scope of the present disclosure. For example, the functions included in each functional part can be rearranged in a logically consistent manner. Multiple functional parts and so forth may be combined into a single part or divided into multiple parts. Further, each embodiment according to the present disclosure described above does not need to be implemented exactly as described in the embodiment, and may be implemented with features having been combined or omitted as appropriate. A variety of variations and amendments to the content of the present disclosure can be made by one skilled in the art based on the present disclosure. Therefore, such variations and amendments are included in the scope of the present disclosure. For example, in each embodiment, each functional part, each means, each step and so on can be added to other embodiments so long as there are no logical inconsistencies, or can be replaced with each functional part, each means, each step, and so on of other embodiments. In each embodiment, a plurality of each functional part, each means, each step, and so on can be combined into a single functional part, means, or step or divided into multiple functional parts, means, or steps. Each of the above-described embodiments of the present disclosure is not limited to faithful implementation of each of the described embodiments, and may be implemented by combining or omitting some of the features as appropriate.

The above-described embodiment is not limited to only being implemented as the electronic device 1. For example, the embodiment described above may be implemented as a method of designing a device such as the electronic device 1.

The electronic device 1 according to the embodiment described above is described as including components that constitute a so-called radar sensor, such as the transmission antenna array 24 and the reception antenna array 31. However, an electronic device according to an embodiment may be implemented with a configuration such as the signal processor 10. In this case, the signal processor 10 may be implemented so as to have a function of processing signals handled by the transmission antenna array 24 and the reception antenna array 31, for example.

REFERENCE SIGNS

| | |
|---|---|
| 1 | electronic device |
| 10 | signal processor |
| 11 | signal generation processor |
| 12 | reception signal processor |
| 13 | time series signal generator |
| 14 | calculating unit |
| 21 | transmission DAC |
| 22 | transmission circuit |
| 23 | millimeter wave transmission circuit |
| 24 | transmission antenna array |
| 31 | reception antenna array |
| 32 | mixer |
| 33 | reception circuit |
| 34 | reception ADC |
| 50 | communication interface |
| 60 | external device |
| 70 | housing |
| 80 | cover part |
| 90 | substrate |
| 200 | object |
| 210 | vibration source (loudspeaker) |
| 310 | reference machine (laser vibrometer) |
| 320 | signal generator |
| 330 | amplifier (audio amplifier) |
| 340 | data logger |

**Claims**

1. An electronic device comprising:

a transmission antenna configured to transmit a transmission wave;
a reception antenna configured to receive a reflection wave resulting from reflection of the transmission wave;
a cover part configured to cover at least part of at least one of the transmission antenna or the reception antenna; and
a signal processor configured to detect an object that reflects the transmission wave based on at least one of a transmission signal transmitted as the transmission wave or a reception signal received as the reflection wave,
wherein at least part of at least one of the transmission wave transmitted by the transmission antenna or the reflection wave received by the reception antenna is transmitted and/or received via the cover part, and
a thickness of the cover part is designed based on magnitude-squared coherence.

2. The electronic device according to claim 1, wherein the signal processor detects a vibration of the object that reflects the transmission wave based on at least one of the transmission signal transmitted as the transmission wave or the reception signal received as the reflection wave.

3. The electronic device according to claim 2, wherein the signal processor detects a heartbeat of a human body or an animal that reflects the transmission wave based on at least one of the transmission signal transmitted as the transmission wave or the reception signal received as the reflection wave.

4. The electronic device according to any one of claims 1 to 3, wherein the thickness of the cover part is a thickness including a component in a direction in which at least part of at least one of the transmission wave transmitted by the transmission antenna or the reflection wave received by the reception antenna is transmitted and/or received via the cover part.

5. The electronic device according to any one of claims 1 to 4, wherein the thickness of the cover part is designed based on magnitude-squared coherence calculated by operating a prescribed vibration source and a prescribed reference machine.

6. The electronic device according to claim 5, wherein the magnitude-squared coherence is calculated based on a time-series signal vector of vibration of the vibration source detected by the reference machine and a power spectral density of the time-series signal vector of the vibration of the vibration source detected by the electronic device 1.

7. The electronic device according to any one of claims 1 to 6, wherein the thickness of the cover part is designed based on averaged magnitude-squared coherence.

8. The electronic device according to any one of claims 1 to 7, wherein at least part of the cover part includes a radome or the cover part includes at least part of a radome.

9. The electronic device according to any one of claims 1 to 8, wherein the thickness of the cover part is designed based also on a result of electromagnetic field analysis.

10. A method for designing an electronic device,

the electronic device including

a transmission antenna configured to transmit a transmission wave,

a reception antenna configured to receive a reflection wave resulting from reflection of the transmission wave,
a cover part configured to cover at least part of at least one of the transmission antenna or the reception antenna, and
a signal processor configured to detect an object that reflects the transmission wave based on at least one of a transmission signal transmitted as the transmission wave or a reception signal received as the reflection wave,

at least part of at least one of the transmission wave transmitted by the transmission antenna or the reflection wave received by the reception antenna being transmitted and/or received via the cover part,
the method comprising:
designing a thickness of the cover part based on magnitude-squared coherence.

11. A method for designing an electronic device,

the electronic device including

a transmission antenna configured to transmit a transmission wave,
a reception antenna configured to receive a reflection wave resulting from reflection of the transmission wave, and
a signal processor configured to detect an object that reflects the transmission wave based on at least one of a transmission signal transmitted as the transmission wave or a reception signal received as the reflection wave,

the method comprising:
designing a prescribed specification in the electronic device based on magnitude-squared coherence.

# FIG. 1

# FIG. 2

# FIG. 3

# FIG. 4

START

DESIGN ANTENNA ARRAYS
AND BASIC STRUCTURE — S11

DESIGN CLEARANCE AND
THICKNESS OF COVER PART — S12

DETERMINE (N) DESIGN CRITERIA — S13

ELECTROMAGNETIC FIELD ANALYSIS — S14

S15

BASIC ABILITY SATISFIED?    N

S16

NEXT DESIGN CRITERION
( n → n + 1 )

Y

EVALUATE ABILITY TO DETECT
VIBRATION USING PROTOTYPES
OF DESIGN CRITERIA — S17

SELECT OPTIMUM DESIGN CRITERIA — S18

END

EP 4 468 025 A1

# FIG. 5

## FIG. 6

EP 4 468 025 A1

FIG. 7

FIG. 8

# FIG. 9

# FIG. 10

EP 4 468 025 A1

# FIG. 11

```
        ┌─────────────┐
        │    START    │
        └──────┬──────┘
               │
               ▼
   ┌───────────────────────┐
   │   PERFORM DESIGN FOR   │─── S21
   │   PRESCRIBED TARGET    │
   └───────────┬───────────┘
               │
               ▼
   ┌───────────────────────┐
   │ DETERMINE (N) DESIGN   │─── S22
   │       CRITERIA         │
   └───────────┬───────────┘
               │                                         ◄──────────┐
               ▼                                                    │
   ┌───────────────────────┐                                       │
   │  DESIGN SIMULATION ETC.│─── S23                                │
   └───────────┬───────────┘                                       │
               │                                                    │
               ▼                              S24                   │
        ╱───────────────────────╲                                  │
       ╱                         ╲      N                           │
      │  BASIC ABILITY SATISFIED? ├──────┐                          │
       ╲                         ╱       │                          │
        ╲───────────┬───────────╱        │              S25         │
                    │ Y                  │                          │
                    │                    ▼                          │
                    │        ┌───────────────────────┐              │
                    │        │  NEXT DESIGN CRITERION │              │
                    │        │     ( n → n + 1 )      ├──────────────┘
                    │        └───────────────────────┘
                    ▼
   ┌───────────────────────┐
   │  EVALUATE ABILITY TO   │
   │ DETECT VIBRATION USING │─── S26
   │ PROTOTYPES OF DESIGN   │
   │       CRITERIA         │
   └───────────┬───────────┘
               │
               ▼
   ┌───────────────────────┐
   │ SELECT OPTIMUM DESIGN  │─── S27
   │       CRITERIA         │
   └───────────┬───────────┘
               │
               ▼
        ┌─────────────┐
        │     END     │
        └─────────────┘
```

## INTERNATIONAL SEARCH REPORT

| International application No. |
| --- |
| **PCT/JP2022/037501** |

### A. CLASSIFICATION OF SUBJECT MATTER

**G01S 7/03**(2006.01)i
FI: G01S7/03 246

According to International Patent Classification (IPC) or to both national classification and IPC

### B. FIELDS SEARCHED

Minimum documentation searched (classification system followed by classification symbols)

G01S 7/00 - G01S 7/42; G01S 13/00 - G01S 13/95; G01N 22/00 - G01N 22/04; G01N 24/00 - G01N 24/14; G01R 33/28 - G01R 33/64; G01V 1/00 - G01V 99/00; H01Q 1/00 - H01Q 1/10; H01Q 1/27 - H01Q 1/52;

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

IEEE Xplore

### C. DOCUMENTS CONSIDERED TO BE RELEVANT

| Category* | Citation of document, with indication, where appropriate, of the relevant passages | Relevant to claim No. |
| --- | --- | --- |
| A | JP 2020-159985 A (MITSUBISHI ELECTRIC CORP.) 01 October 2020 (2020-10-01) entire text, all drawings | 1-11 |
| A | JP 2016-145777 A (DENSO CORP.) 12 August 2016 (2016-08-12) entire text, all drawings | 1-11 |
| A | JP 2004-301592 A (TOYOTA MOTOR CORP.) 28 October 2004 (2004-10-28) entire text, all drawings | 1-11 |
| A | JP 2002-31680 A (TOTO LTD.) 31 January 2002 (2002-01-31) entire text, all drawings | 1-11 |
| A | US 2021/0349138 A1 (PERISENS GMBH) 11 November 2021 (2021-11-11) entire text, all drawings | 1-11 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 November 2022** | **06 December 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 468 025 A1

<div align="center">

### INTERNATIONAL SEARCH REPORT
**Information on patent family members**
</div>

International application No.

**PCT/JP2022/037501**

| Patent document cited in search report | | | Publication date (day/month/year) | Patent family member(s) | | | Publication date (day/month/year) |
|---|---|---|---|---|---|---|---|
| JP | 2020-159985 | A | 01 October 2020 | (Family: none) | | | |
| JP | 2016-145777 | A | 12 August 2016 | US | 2016/0231417 | A1 | |
| JP | 2004-301592 | A | 28 October 2004 | (Family: none) | | | |
| JP | 2002-31680 | A | 31 January 2002 | (Family: none) | | | |
| US | 2021/0349138 | A1 | 11 November 2021 | WO | 2020/074667 | A1 | |
| | | | | CN | 112840225 | A | |

Form PCT/ISA/210 (patent family annex) (January 2015)

30

**REFERENCES CITED IN THE DESCRIPTION**

**Patent documents cited in the description**

- JP 2022006696 A
- JP 2002071825 A
- JP 2021032880 A
- JP 2012093305 A